(19) Europäisches Patentamt
European Patent Office
Office européen des brevets

(11) Publication number: 0 255 377
A2

# (12) EUROPEAN PATENT APPLICATION

(21) Application number: 87306738.3

(22) Date of filing: 30.07.87

(51) Int. Cl.⁴: C 12 N 15/00
C 12 N 5/00, A 01 H 1/00
// C07K13/00, A01H5/10

(30) Priority: 31.07.86 US 891529 28.07.87 US 78924

(43) Date of publication of application:
03.02.88 Bulletin 88/05

(84) Designated Contracting States:
AT BE CH DE ES FR GB GR IT LI LU NL SE

(71) Applicant: CALGENE, INC.
1920 Fifth Street Suite F.
Davis California 95616 (US)

(72) Inventor: Kridl, Jean C.
538 Reed Drive
Davis California 95616 (US)

Knauf, Vic C.
2454 Elendil Lane
Davis California 95616 (US)

(74) Representative: Paget, Hugh Charles Edward et al
MEWBURN ELLIS & CO. 2/3 Cursitor Street
London EC4A 1BQ (GB)

## (54) Acyl carrier protein- DNA sequence and synthesis.

(57) DNA sequences are provided coding for acyl carrier protein, which sequence can be used for production of acyl carrier protein as an end product or in plant seed to enhance seed oil production. A regulated promoter is provided which substantially limits expression of the acyl carrier protein to seed tissue.

EP 0 255 377 A2

## Description

# ACYL CARRIER PROTEIN - DNA SEQUENCE AND SYNTHESIS

FIELD OF THE INVENTION

Acyl carrier protein is expressed under conditions where the protein may be isolated for in vitro use or the protein is intracellularly translocated to a chloroplast or related organelle for modification of fatty acid production in vivo. Constructs are provided which allow for expression of acyl carrier protein in seed tissue using a seed specific promoter.

BACKGROUND OF THE INVENTION

Plants provide a rich source of a variety of products which find use in foods, as raw materials, and as finished products. Vegetable fatty acids find extensive use for a wide variety of commercial purposes, being used as vegetable oils for cooking, as lubricants, in alkyd resins, as specialty chemicals, and the like. For the most part, the plant fatty acids tend to be of 18 carbon atoms, there usually being only a minor level of fatty acids having fewer than 16 carbon atoms. For many purposes, it would be desirable to have fatty acids in the range of 8 to 14 carbon atoms. There is, therefore, substantial interest in developing methods for producing vegetable oils where there is a substantial proportion of the total fatty acids of 14 carbon atoms or fewer.

To achieve this purpose, it will be necessary to modify the constituent members of the metabolic pathway resulting in the formation of fatty acids and their elongation to higher fatty acids. Toward this purpose, it will be necessary to be able to produce one or more components along the fatty acid metabolic chain which modify the course of the plant metabolism. In addition, there may be significant commercial applications for individual components of the fatty acid metabolic pathway.

BRIEF DESCRIPTION OF THE RELEVANT LITERATURE

Kuo and Ohlrogge, Archives of Biochem. and Biophys. (1984) 234:290-296, describe the primary structure of spinach acyl carrier protein. Ohlrogge and Kuo, J. Biol. Chem. (1985) 260:8032-8037 report the existence of different isoforms of acyl carrier protein expressed differently in different tissues. Crouch et al., J. Mol. Appl. Genet. (1983) 2:273-283, report the synthesis of cDNA coding for napin protein.

SUMMARY OF THE INVENTION

DNA constructs are provided which provide for expression of plant acyl carrier protein. Particular constructs are produced which employ transcription initiation regions resulting in expression in plant embryos during seed maturation. The composition and amount of fatty acid can be modulated by modifying the constituents in the chloroplast or related organelle involved in a metabolic pathway in the production of fatty acids.

DESCRIPTION OF SPECIFIC EMBODIMENTS

Methods and compositions are provided for production of acyl carrier protein as an end product for use in vitro or in conjunction with seed formation of plants to provide for modified expression of fatty acids in vivo. Towards this end, DNA constructs are prepared, where the sequence encoding plant acyl carrier protein is joined to transcriptional initiation and termination regulatory regions, which are func tional in a predetermined host for expression of the acyl carrier protein.

The expression constructs provide in the 5′-3′ direction of transcription, a transcriptional initiation regulatory region, either constitutive or regulated, an open reading frame coding for at least a functional portion of the acyl carrier protein, desirably including a transit peptide sequence providing for translocation to the chloroplast for in vivo use, and a transcriptional termination regulatory region functional in the appropriate host.

Depending upon the host, the regulatory regions will vary. For expression in a prokaryotic or eukaryotic microorganism, particularly unicellular, host, a wide variety of constitutive or regulatable, promoters may be employed. In these instances, the primary purpose for the preparation of the acyl carrier protein is the use of the acyl carrier protein for in vitro applications.

For the most part, the constructs will involve regulatory regions functional in plants which provide for enhanced production of acyl carrier protein for enhanced and/or modification of the fatty acid composition.

The coding sequence which is employed may be derived from natural sources, synthesized, or combinations thereof. To obtain the gene from a natural source, any of a variety of plants or bacteria may be used as the source of the gene. Plants include spinach, Brassica, e.g. campestris or napus, coconut, cotton, safflower, sunflower, Cuphea etc. Among various ways in which the gene may be obtained, a library may be prepared, either genomic or cDNA. Probes may be prepared based on the amino acid sequence of the acyl carrier protein. Since it is found that there is a substantial immunological cross-reactivity between acyl carrier proteins from different sources, both prokaryotic and eukaryotic polyclonal antibodies may be employed for isolating acyl carrier proteins from a particular source and may be further used to isolate acyl carrier proteins from other plant sources. The acyl carrier protein may then be sequenced in whole or in part, and probes designed based on the peptide sequence. Where only a partial DNA sequence is obtained, the partial

sequence may be satisfactory or the gene may be walked, so as to ensure that the entire coding sequence has been obtained.

Once the desired sequence has been obtained, it may be manipulated in a variety of ways. Where the sequence involves non-coding flanking regions, the flanking regions may be subjected to resection, using, for example, a nuclease such as Bal 31, restriction with a restriction endonuclease, or modification by employing in vitro mutagenesis, primer repair, or other methods for introducing mutations or lesions into the sequence. Thus, transitions, tranversions, deletions, and insertions may be performed on the naturally occurring sequence. In addition, all or a portion of the sequence may be synthesized, where one or more codons may be modified to provide for a modified amino acid sequence, or one or more codon mutations may be introduced to provide for a convenient restriction site or other purpose involved with construction or expression. The gene may be further modified by employing synthetic adaptors, linkers to introduce one or more convenient restriction sites, or the like.

The acyl carrier protein may be any one of the isozymes which may be found in a particular host, such as ACP-I and ACP-II as designated in Ohlrogge and Kuo, supra, as found in spinach, or their analogues as found in other plant hosts.

Of particular interest is the spinach acyl carrier protein, more particularly ACP-I which has the following sequence.

Spinach ACP-I

```
a)   1 AACTTAATATTCTTACTCAGGATAAGCTTCTCACTCTCTCTCTCTCTCTCTTACTACC  60

a)  61 ATG GCT TCC TTG TCC GCG ACG ACA ACA GTT AGG GTT CAA CCA TCG  105
b)     MET Ala Ser Leu Ser Ala Thr Thr Thr Val Arg Val Gln Pro Ser

a) 106 TCT TCG TCT CTA CAT AAA CTT AGC CAG GGG AAT GGA AGA TGT TCA  150
b)     Ser Ser Ser Leu His Lys Leu Ser Gln Gly Asn Gly Arg Cys Ser

a) 151 AGT ATA GTG TGT TTG GAT TGG GGC AAA AGC AGT TTC CCA ACA CTC  195
b)     Ser Ile Val Cys Leu Asp Trp Gly Lys Ser Ser Phe Pro Thr Leu

a) 196 AGG ACT TCT CGT CGT CGA TCC TTT ATC TCT GCT GCA AAG AAG GAA  240
b)     Arg Thr Ser Arg Arg Arg Ser Phe Ile Ser Ala Ala Lys Lys Glu
c)                                             Ala Lys Lys Glu

a) 241 ACA ATT GAC AAA GTG TGC GAC ATT GTA AAG GAG AAA CTG GCT TTA  285
b)     Thr Ile Asp Lys Val Cys Asp Ile Val Lys Glu Lys Leu Ala Leu
c)     Thr Ile Asp Lys Val Ser Asp Ile Val Lys Glu Lys Leu Ala Leu
                           *
a) 286 GGA GCT GAT GTT GTG GTC ACA GCT GAT TCC GAG TTT AGT AAA CTC  330
b)     Gly Ala Asp Val Val Val Thr Ala Asp Ser Glu Phe Ser Lys Leu
c)     Gly Ala Asp Val Val Val Thr Ala Asp Ser Glu Phe Ser Lys Leu

a) 331 GGT GCT GAT TCA TTG GAC ACG GTT GAG ATA GTG ATG AAC CTC GAG  375
b)     Gly Ala Asp Ser Leu Asp Thr Val Glu Ile Val Met Asn Leu Glu
c)     Gly Ala Asp Ser Leu Asp Thr Val Glu Ile Val Met Asn Leu Glu

a) 376 GAA GAG TTC GGT ATC AAT GTG GAT GAA GAT AAA GCT CAA GAT ATA  420
b)     Glu Glu Phe Gly Ile Asn Val Asp Glu Asp Lys Ala Gln Asp Ile
c)     Glu Glu Phe Gly Ile Asn Val Asp Glu Asp Lys Ala Gln Asp Ile

a) 421 TCA ACC ATC CAA CAA GCC GCC GAC GTT ATT GAG AGT CTT CTT GAG  465
b)     Ser Thr Ile Gln Gln Ala Ala Asp Val Ile Glu Ser Leu Leu Glu
c)     Ser Thr Ile Gln Gln Ala Ala Asp Val Ile Glu (-) Leu Leu Glu
                                                   *
a) 466 AAG AAA TAG ACCCCAAACTATGCTCTCTCAATCAGTCAATCACCTAGCTAGGGAAT  521
b)     Lys Lys ---
c)     Lys Lys Ala-COOH
               *

a) 522 GAATTC
```

a) cDNA sequence
b) amino acid sequence predicted by DNA sequence
c) amino acid sequence determined by protein sequencing (Kuo and Ohlrogge, 1984, supra)
* differences between b) and c)

The sequences of interest will generally have at least 300 bp usually at least about 360 bp, and desirably 411 base pairs, where the entire coding sequence includes 411 base pairs. In addition, there may be both 5′ and 3′

non-coding flanking regions which may extend from 1 bp to 200 bp or more from the 5′ or 3′ terminus of the coding region, there usually being fewer than about 100 bp, preferably fewer than about 10 bp 5′ of the initiation codon, of the naturally occurring non-coding flanking region.

The open reading frame, coding for the acyl carrier protein or functional fragment thereof will be joined at its 5′ end to a transcriptional initiation regulatory region. Numerous transcriptional initiation regulatory regions are available, which provide for a wide variety of constitutive or regulatable, e.g. inducible, transcription of the structural gene. Depending upon the host, transcriptional initiation regulatory regions may include regions from structural genes from viral, plasmid or, chromosomal genes, or the like. Among transcriptional initiation regions which have been described are regions from bacterial and yeast hosts, such as E. coli, Bacillus subtilis, Saccharomyces cerevisiae, including genes such as β-galactosidase, lambda left and right promoters, glycolytic enzyme promoters, and the like. Among transcriptional initiation regions used for plants are such regions associated with the structural genes for nopaline, octopine, mannopine, ribulose-1,3-bisphosphate carboxylase, the large and small subunits, the full length promoter from cauliflower mosaic virus, napin, phaseolin, etc.

Of particular interest are those promoters which are regulated during seed maturation, particularly those synthesized in cotyledons of the embryo. These regulatory regions include regulatory regions of genes such as napin, phaseolin and glycinin. Napin regulatory regions of particular interest are from Brassica species, more particulary campestris and napus. The regulatory region will generally be at least about 150 bp and not more than about 3500 bp usually not more than about 2500 bp, and desirably not more than about 1000 bp. The napin gene has been described, Crouch, et al., supra, although there has been no disclosure of the regulatory region, nor the use of the regulatory region for an heterologous gene.

The transcriptional initiation regulatory region and coding region may be joined directly, where there are convenient restriction sites for the two regions or such restriction site(s) have been introduced, or, as appropriate, by means of synthetic adaptors or linkers. A number of regulatory regions are available as plasmids where the initiation and termination regulatory regions are separated by a polylinker, so that a number of restriction sites are available for insertion of the structural gene. These expression constructs are mostly available for microorganism hosts.

While a number of transcriptional initiation and termination regions functional in plants have been isolated, particularly from genes on the Ti- and Ri-plasmids, these regions have not achieved the level of readily available constructs including polylinkers, markers, replication systems and the like. Furthermore, for the present invention there is primary interest for expression to be regulated so that transcription is initiated in seed. For this purpose, a gene such as the napin gene is of substantial interest.

A napin regulatory region can be obtained by employing a probe which comprises a sequence adjacent the 3′- or 5′-terminus or intermediate coding sequence of the structural gene for screening a genomic library of the napin host, in the case of Crouch, 1983, supra, the rapeseed host. By identifying fragments which hybridize under stringent conditions with the probe, fragments having the napin structural gene may be identified. Potential regulatory sequences 5′ of the napin structural gene can be identified by restriction mapping and DNA sequence analysis. These sequences can be manipulated under various conditions to remove in whole or in part the codons coding for napin, leaving the uncoded 5′ region free or substantially free of the napin coding region. In some instances, it may be desirable to remove a short non-coding region adjacent the initiation codon, usually fewer than about 20 bp, more usually fewer than about 10 bp. For further details, the experimental section should be consulted.

After joining of the open reading frame for the acyl carrier protein structural gene and the transcriptional initiation regulatory region, a functional transcriptional termination regulatory region may be present, which has been included as a result of the method for construction or one may be introduced. The termination region may be from the same structural gene as the initiation region, acyl carrier protein gene, as is convenient. The termination region will usually include a terminator and sequence coding for polyadenylation.

The gene may naturally include or be modified by introducing a signal sequence for intracellular translocation, particularly to the leucoplast in seed or the chloroplast in other plant cells.

In developing the expression construct, the various components of the expression construct or fragments thereof will normally be inserted into a convenient cloning vector which is capable of replication in a bacterial host, e.g., E. coli. Numerous vectors exist that have been described in the literature. After each cloning, the plasmid may be isolated and subjected to further manipulation, such as restriction, insertion of new fragments, ligation, deletion, resection, insertion, in vitro mutagenesis or primer repair, so as to tailor the components to the desired sequence. Once the construct has been completed, it may then be transfered to an appropriate vector for further manipulation in accordance with the manner of transformation of the plant cell.

Normally, included with the expression construct will be a structural gene having the necessary regulatory regions for expression in a host and providing for selection of transformant cells. The gene may provide for resistance to a cytotoxic agent, e.g. antibiotic, heavy metal, toxin, etc., complementation providing phototrophy to an auxotrophic host, viral immunity or the like. Depending upon the number of different host species the expression construct or components thereof are introduced, one or more markers may be employed, where different conditions for selection are used for the different hosts.

The manner in which the construct is introduced into the plant host is not critical to this invention. Any method which provides for efficient transformation may be employed. Various methods include the use of Ti- or Ri-plasmids, microinjection, electroporation, liposome fusion, or the like. In many instances, it will be

desirable to have the construct bordered on one or both sides by T-DNA, particularly having the left and right borders, more particularly the right border. This is particularly useful when the construct uses A. tumefaciens or A. rhizogenes as a mode for transformation, although the T-DNA borders may find use with other modes of transformation.

Where the Agrobacterium is used for plant cell transformation, a vector may be used which may be introduced into the Agrobacterium host for homologous recombination with T-DNA of the Ti- or Ri-plasmid present in the Agrobacterium host. The Ti- or Ri- plasmid containing the T-DNA for recombination may be armed (capable of causing gall formation) or disarmed (incapable of causing gall formation), the latter being permissible, so long as the vir genes are present in the transformed host. The armed plasmid can give a mixture of normal plant cell and gall.

In some instances where Agrobacterium is used as the vehicle for transforming plant cells, the expression construct bordered by the T-DNA border(s) will be inserted into a broad host spectrum vector, there being broad host spectrum vectors described in the literature. Commonly used in pRK2 or derivatives thereof. See, for example, Ditta et al. (1980) PNAS USA, 77:7347-7351 and EPA 0 120 515, which are incorporated herein by reference. Included with the expression construct and the T-DNA will be one or more markers, which allow for selection of transformed Agrobacterium and transformed plant cells. A number of markers have been developed for use with plant cells, such as resistance to chloramphenicol, the aminoglycoside G418, hygromycin, or the like. The particular marker employed is not essential to this invention, one or another marker being preferred depending on the particular host and the manner of construction.

The expression constructs may be employed with a wide variety of plant life, particularly plant life involved in the production of vegetable oils. These plants include Brassica e.g. napus and campestris, sunflower, safflower, cotton, Cuphea, soybean, and corn.

For transformation of plant cells using Agrobacterium, explants may be combined and incubated with the transformed Agrobacterium for sufficient time for transformation, the bacteria killed, and the plant cells cultured in an appropriate selective medium. Once callus forms, shoot formation can be encouraged by employing the appropriate plant hormones in accordance with known methods and the shoots transferred to rooting medium for regeneration of plants. The plants may then be grown to seed and the seed used to establish repetitive generations and for isolation of vegetable oils.

The DNA sequences can also be used as probes for searching for acyl carrier proteins in hosts other than the host from which the gene was derived. In addition, the acyl carrier protein produced in accordance with the subject invention can be used in preparing antibodies for assays for detecting acyl carrier protein. The acyl carrier protein can also be used in conjunction with chloroplast lysates to enhance the production and/or modify the composition of the fatty acids prepared in vitro. The acyl carrier protein can also be used for studying the mechanism of fatty acid formation in plants and bacteria.

The following examples are offered by way of illustration and not by way of limitation.

## EXPERIMENTAL

## MATERIALS AND METHODS

### Cloning Vectors

Cloning vectors used include the pUC vectors, pUC8 and pUC9 (Vieira and Messing, 1982) Gene, 19:259-268; pUC18 and pUC19 (Norrander et al., 1983) Gene, 26:101-106, Yanisch-Perron et al., (1985) Gene, 33:103-119, analogous vectors exchanging chloramphenicol resistance (CAM) as a marker for the ampicillin resistance of the pUC plasmids described above (pUC-CAM [pUC12-Cm, pUC13-Cm] Buckley, K., Ph.D. Thesis, U.C.S.D., CA 1985). The multiple cloning sites of pUC18 and pUC19 vectors were exchanged with those of pUC-CAM to create pCGN565 and pCGN566 which are CAM resistant. Also used were pUC118 and pUC119, which are respectively, pUC18 and pUC19 with the intergenic region of M13, from a HgiAI site at 5465 to the AhaIII site at 5941, inserted at the NdeI site of pUC. (Available from Vieira J. and Messing, J. Waksman Institute, Rutgers University, Rutgers, N.J.).

### Materials

Terminal deoxynucleotide transferase (TDT), RNaseH, E. Coli, DNA polymerase, T4 kinase, and restriction enzymes were obtained from Bethesda Research Laboratories; E. coli DNA ligase was obtained from New England Biolabs; reverse transcriptase was obtained from Life Sciences, Inc.; isotopes were obtained from Amersham; X-gal was obtained from Bachem, Inc. Torrance, CA.

### Construction of cDNA Library from Spinach Leaves

Total RNA was extracted from young spinach leaves in 4M guanidine thiocyanate buffer as described by Facciotti et al. (Biotechnology (1985) 3:241-246.). Total RNA was subjected to oligo(dT)-cellulose column chromatography two times to yield poly(A)+ RNA as described by Maniatis et al., Molecular Cloning: A Laboratory Manual, Cold Spring Harbor Laboratory, New York (1982). A cDNA library was constructed in pUC13-Cm according to the method of Gubler and Hoffman, (Gene (1983) 25:263-269) with slight modifications. RNasin was omitted in the synthesis of first strand cDNA as it interfered with second strand cDNA synthesis if not completely removed, and dCTP was used to tail the vector DNA and dGTP to tail

double-stranded cDNA instead of the reverse as described in the paper. The annealed cDNA was transformed to competent E. coli JM83 (Messing in Recombinant DNA Technical Bulletin, NIH Publication No. 79-99, 2 (1979) No.2:43-48.) cells according to Hanahan (J. Mol. Biol. (1983) 166:557-580) and spread onto LB agar plates (Miller, Experiments in Molecular Genetics (1972) Cold Spring Harbor Laboratory, Cold Spring Harbor, New York) containing 50μg/ml chloramphenicol and 0.005% X-Gal.

Identification of Spinach ACP-I cDNA

A total of approximately 8000 cDNA clones were screened by performing Southern blots (Southern J. Mol. Biol (1975) 98:503) and dot blot (described below) hybridizations with clone analysis (see below) DNA from 40 pools representing 200 cDNA clones each. A 5′ end-labeled synthetic oligonucleotide (ACPP4) that is at least 66% homologous with a 16 amino acid region of spinach ACP-I 5′-CATGTCTTGAGCCTTGTCCTCATCCACATTGATACCAAACTCCTCCTC-3′ is the complement to a DNA sequence that could encode the 16 amino acid peptide glu-glu-glu-phe-gly-ile-asn-val-asp-glu-asp-lys-ala-gln-asp-ile, residues 49-64 of spinach ACP-I (Kuo and Ohlrogge Arch. Biochem. Biophys. (1984) 234:290-296) and was used for an ACP proble.

Clone analysis DNA for Southern and dot blot hybridizations was prepared as follows. Transformants were transferred from agar plates to LB containing 50μg/ml chloramphenicol in groups of ten clones per 10/ml media. Cultures were incubated overnight in a 37° C shaking incubator and then diluted with an equal volume of media and allowed to grow for 5 more hours. Pools of 200 cDNA clones each were obtained by mixing contents of 20 samples. DNA was extracted from these cells as described by Birnbolm and Doly (Nucleic Acids Res. (1979) 7:1513-1523). DNA was purified to enable digestion with restriction enzymes by extractions with phenol and chloroform followed by ethanol precipitation. DNA was resuspended in sterile, distilled water and 1μg of each of the 40 pooled DNA samples was digested with EcoRI and HindIII and electrophoresed through 0.7% agarose gels. DNA was transferred to nitrocellulose filters following the blot hybridization technique of Southern.

ACPP4 was 5′ end-labeled using γ-$^{32}$P dATP and T4 kinase according to the manufacturer's specifications. Nitrocellulose filters from Southern blot transfer of clone analysis DNA were hybridized (24 hours, 42° C) and washed according to Berent et al. (BioTechniques (1985) 3:208-220). Dot blots of the same set of DNA pools were prepared by applying 1μg of each DNA pool to nylon membrane filters in 0.5M NaOH. These blots were hybridized with the probe for 24 hours at 42° C in 50% formamide/1% SDS/1 M NaCl, and washed at room temperature in 2X SSC/0.1% SDS (1X SSC = 0.15M NaCl; 0.015M Na citrate; SDS-sodium dodecylsulfate). DNA from the pool which was hybridized by the ACPP4 oligoprobe was transformed to JM83 cells and plated as above to yield individual transformants. Dot blots of these individual cDNA clones were prepared by applying DNA to nitrocellulose filters which were hybridized with the ACPP4 oligonucleotide probe and analyzed using the same conditions as for the Southern blots of pooled DNA samples.

Nucleotide Sequence Analysis

The positive clone, pCGN1SOL, was analyzed by digestion with restriction enzymes and the following partial map was obtained.

```
pUC13-Cm    |-35-|     248    |-63-|    152    |  ~200   ~|
 ........ *________________________________________________* ........
       |    |     |           |    |           |          |
       H    H     N           P    Xh          E          SXBSmSsE**

   H-HindIII     N-NcoI     P-PvuII     Xh-XhoI
   E-EcoRI       S-SalI     X-XbaI      Sm-SmaI
   B-BamHI       Ss-SstI    *-former PstI site destroyed
                              with tailing

   **polylinker with available restriction sites indicated
```

The cDNA clone was subcloned into pUC118 and pUC119 using standard laboratory techniques of restriction, ligation, transformation, and analysis (Maniatis et al., (1982) supra). Single-stranded DNA template was prepared and DNA sequence was determined using the Sanger dideoxy technique (Sanger et al., Proc. Nat. Acad. Sci. (1977) USA 74:5463-5467). Sequence analysis was performed using a software package from IntelliGenetics, Inc.

pCGN1SOL contains an (approximately) 700 bp cDNA insert including a stretch of A residues at the 3′ terminus which represents the poly(A) tail of the mRNA. An ATG codon at position 61 is presumed to encode the MET translation initiation codon. This codon is the start of a 411 nucleotide open reading frame, of which, nucleotides 229-471 encode a protein whose amino acid sequence corresponds almost perfectly with the published amino acid sequence of ACP-I of Ohlrogge and Kuo supra sequence as described previously. Discrepancies between the two amino acid sequences are indicated in the sequence set forth previously. In addition to mature protein, the pCGN1SOL also encodes a 56 residue transit peptide sequence, as might be expected for a nuclear-encoded chloroplast protein.

Construction of a Napin Promoter

There are 298 nucleotides upstream of the ATG start codon of the napin gene on the pgN1 clone (a 3.3 kb EcoRI fragment of B. napus genomic DNA containing a napin gene cloned into pUC8, available from Marti Crouch, University of Indiana). pgN1 DNA was digested with EcoRI and SstI and ligated to EcoRI/SstI digested pCGN706. (pCGN706 is an XhoI/PstI fragment containing 3′ and polyadenylation sequences of another napin cDNA clone pN2 (Crouch et al., 1983 supra) cloned in pCGN566 at the SalI and PstI sites.) The resulting clone pCGN707 was digested with SalI and treated with the enzyme Bal31 to remove some of the coding region of the napin gene. The resulting resected DNA was digested with SmaI after the Bal31 treatment and religated. One of the clones pCGN713, selected by size, was subcloned by EcoRi and BamHI digestion into both EcoRI/BamHI digested pEMBL18 (Dente et al., Nucleic Acids Res. (1983) 11:1645-1655) and pUC118 to give E418 and E4118 respectively. The extent of Bal31 digestion was confirmed by Sanger dideoxy sequencing of E418 template. The Bal31 deletion of the promoter region extended only to 57 nucleotides downstream of the start codon, thus containing the 5′ end of the napin coding sequence and about 300 bp of the 5′ non-coding region, E4118 was tailored to delete all of the coding region of napin including the ATG start codon by in vitro mutagenesis by the method of Zoller and Smith (Nucleic Acids Res. (1982) 10:6487-6500) using an oligonucleotide primer 5′-GATGTTTTGTATGTGGGCCCCTAGGAGATG-3′. Screening for the appropriate mutant was done by two transformations into E. coli strain JM83 (Messing, J., supra) and SmaI digestion of putative transformants. The resulting napin promoter clone is pCGN778 and contains 298 nucleotides from the EcoRI site of pgN1 to the A nucleotide just before the ATG start codon of napin. The promoter region was subcloned into a chloramphenicol resistant background by digestion with EcoRI and BamHI and ligation to EcoRI/BamHI digested pCGN565 to give pCGN779c.

Extension of the Napin Promoter Clone

pCGN779c contains only 298 nucleotides of potential 5′-regulatory sequence. The napin promoter was extended with 1.8 kb fragment found upstream of the 5′-EcoRI site on the original λBnNa clone. The ~3.5 kb XhoI fragment of λBnNa (available from M. Crouch), which includes the napin region, was subcloned into SalI-digested pUC119 to give pCGN930. A HindIII site close to a 5′ XhoI site was used to subclone the HindIII/EcoRI fragment of pCGN930 into HindIII/EcoRI-digested Bluescript + (Vector Cloning Systems, San Diego, CA) to give pCGN942. An extended napin promoter was made by ligating pCGN779c digested with EcoRI and PstI and pCGN942 digested with EcoRI and PstI to make pCGN943. This promoter contains ~2.1 kb of sequence upstream of the original ATG of the napin gene contained on λBnNa.

Napin Cassette

The extended napin promoter and a napin 3′-regulatory region is combined to make a napin cassette for expressing genes seed-specifically. The napin 3′ region used is from the plasmid pCGN1924 containing the XhoI/EcoRI fragment from pgN1 (XhoI site is located 18 nucleotides from the stop codon of the napin gene) subcloned into EcoRI/SalI digested pCGN565. HindIII/PstI digested pCGN943 and pCGN1924 are ligated to make the napin cassette pCGN944, with unique cloning sites SmaI, SalI and PstI for inserting genes.

Napin - ACP Construct

pCGN796 was constructed by ligating pCGN1SOL digested with HindIII/BamHI, pUC8 digested with HindIII and BamHI and pUC118 digested with BamHI. The ACP gene from pCGN796 was transferred into a chloramphenicol background by digestion with BamHI and ligation with BamHI digested pCGN565. The resulting pCGN1902 was digested with EcoRI and SmaI and ligated to EcoRI/SmaI digested pUC118 to give pCGN1920. The ACP gene in pCGN1920 was digested at the NcoI site, filled in by treatment with the Klenow fragment, digested with SmaI and religated to form pCGN1919. This eliminated the 5′-coding sequences from the ACP gene and regenerated the ATG. This ACP gene was flanked with PstI sites by digesting pCGN1919 with EcoRI, filling in the site with the Klenow fragment and ligating a PstI linker. This clone is called pCGN945. The ACP gene of pCCGN945 was moved as a BamHI/PstI fragment to pUC118 digested with BamHI and PstI to create pCGN945a so that a SmaI site (provided by the pUC118) would be at the 5′-end of the ACP sequence to facilitate cloning into the napin cassette pCGN944. pCGN945a digested with SmaI and PstI was ligated to pCGN944 digested with SmaI and PstI to produce the napin ACP cassette pCCGN946. The napin ACP cassette was then transferred into the binary vector pCGN783 by cloning from the HindIII site to produce pCGN948.

Construction of pCGN783

pCGN783 is a binary plasmid containing the left and right T-DNA borders of A. tumefaciens (Barker et al. Plant Mol. Biol. (1983) 2:335-350); the gentamicin resistance gene of pPH1JI (Hirsch et al., (1984) Plasmid 12, 139-141) the 35S promoter of cauliflower mosaic virus (CaMV) (Gardner et al., Nucleic Acids Res. (1981) 9:2871-2890), the kanamycin resistance gene of Tn5 (Jorgensen et al., infra and Wolff et al. ibid (1985) 13:355-367) and the 3′ region from transcript 7 of pTiA6 (Barker et al., (1983) supra).

To obtain the gentamicin resistance marker, the gentamicin resistance gene was isolated from a 3.1 kb EcoRI-PstI fragment of pPHIJ1 and cloned into pUC9 yielding pCGN549. The HindIII-BamHI fragment containing the gentamicin resistance gene was substituted for the HindIII-BglII fragment of pCGN587 creating pCGN594.

pCGN587 was prepared as follows: The HindIII-SmaI fragment of Tn5 containing the entire structural gene for APHII (Jorgensen et al., Mol. gen. Genet. (1979) 177:65) was cloned into pUC8 (Vieira and Messing, Gene (1982) 19:259), converting the fragment into a HindIII-EcoRI fragment, since there is an EcoRI site immediately adjacent to the SmaI site. The PstI-EcoRI fragment containing the 3′-portion of the APHII gene was then combined with an EcoRI-BamHI-SalI-PstI linker into the EcoRi site of pUC7 (pCGN546W). Since this construct does not confer kanamycin resistance, kanamycin resistance was obtained by inserting the Bg1II-pstI fragment of the APHII gene into the BamHI-PstI site (pCGN546X). This procedure reassembles the APHII gene, so that EcoRI sites flank the gene. An ATG codon was upstream from and out of reading frame with the ATG initiation codon of APHII. The undesired ATG was avoided by inserting a Sau3A-PstI fragment from the 5′-end of APHII, which fragment lacks the superfluous ATG, into the BamHI-PstI site of pCGN546W to provide plasmid pCGN550.

The EcoRI fragment containing the APHII gene was then cloned into the unique EcoRI site of pCGN451, which contains an octopine synthase cassette for expression, to provide pCGN552 (1ATG).

pCGN451 includes an octopine cassette which contains about 1556 bp of the 5′ non-coding region fused via an EcoRI linker to the 3′ non-coding region of the octopine synthase gene of pTiA6. The pTI coordinates are 11,207 to 12,823 for the 3′ region and 13,643 to 15,208 for the 5′ region as defined by Barker et al., Plant Mol. Biol. (1983) 2:325.

The 5′ fragment was obtained as follows. A small subcloned fragment containing the 5′ end of the coding region, as a BamHI-EcoRI fragment was cloned in pBR322 as plasmid pCGN407. The BamHI-EcoRi fragment has an XmnI site in the coding region, while pBR322 has two XmnI sites. pCGN407 was digested with XmnI, resected with Ba131 nuclease and EcoRI linkers added to the fragments. After EcoRI and BamHI digestion, the fragments were size fractionated, the fractions cloned and sequenced. In one case, the entire coding region and 10 bp of the 5′ non-translated sequences had been removed leaving the 5′ non-transcribed region, the mRNA cap site and 16 bp ofthe 5′ non-translated region (to a BamHI site) intact. This small fragment was obtained by size fractionation on a 7% acrylamide gel and fragments approximately 130 bp long eluted.

This size fractionated DNA was ligated into M13mp9 and several clones sequenced and the sequence compared to the known sequence of the octopine synthase gene. The M13 construct was designated p14, which plasmid was digested with BamHI and EcoRI to provide the small fragment which was ligated to a XhoI to BamHI fragment containing upstream 5′ sequences from pTiA6 (Garfinkel and Nester, J. Bacteriol. (1980) 144:732) and to an EcoRI to XhoI fragment containing the 3′ sequences.

The resulting XhoI fragment was cloned into the XhoI site of a pUC8 derivative, designated pCGN426. This plasmid differs from pUC8 by having the sole EcoRI site filled in with DNA polymerase I, and having lost the PstI and HindIII site by nuclease contamination of HincII restriction endonuclease, when a XhoI linker was inserted into the unique HincII site of pUC8. The resulting plasmid pCGN451 has a single EcoRI site for the insertion of protein coding sequences between the 5′ non-coding region (which contains 1,550 bp of 5′ non-transcribed sequence including the right border of the T-DNA, the mRNA cap site and 16 bp of 5′ non-translated sequence) and the 3′ region (which contains 267 bp of the coding region, the stop codon, 196 bp of 3′ non-translated DNA, the polyA site and 1,153 bp of 3′ non-transcribed sequence). pCGN451 also provides the right T-DNA border.

The resulting plasmid pCGN451 having the ocs 5′ and the ocs 3′ in the proper orientation was digested with EcoRI and the EcoRI fragment from pCGN551 containing the intact kanamycin resistance gene inserted into the EcoRI site to provide pCGN552 having the kanamycin resistance gene in the proper orientation.

This ocs/KAN gene was used to provide a selectable marker for the trans type binary vector pCGN587.

The 5′ portion of the engineering octopine synthase promoter cassette consists of pTiA6 DNA from the XhoI at bp 15208-13644 (Barker's numbering), which also contains the T-DNA boundary sequence (border) implicated in T-DNA transfer. In the plasmid pCGN587, the ocs/KAN gene from pCGN552 provides a selectable marker as well as the right border. The left boundary region was first cloned in M13mp9 as a HindIII-SmaI piece (pCGN502) (base pairs 602-2213) and recloned as a KpnI-EcoRI fragment in pCGN565 to provide pCGN580. pCGN565 is a cloning vector based on pUC8-Cm, but containing pUC18 linkers. pCGN580 was linearized with BamHI and used to replace the smaller Bg1II fragment of pVCK102 (Knauf and Nester, Plasmid (1982) 8:45), creating pCGN585. By replacing the smaller Sa1I fragment of pCGN585 with the XhoI fragment from pCGN552 containing the ocs/KAN gene, pCGN587 was obtained.

The pCGN594 HindIII-BamHI region, which contains an 5′-ocs-kanamycin-ocs-3′ (ocs is octopine synthase with 5′ designating the promoter region and 3′ the terminator region, see U.S. application serial no. 775,923, filed September 13, 1985) fragment was replaced with the HindIII-BamHI polylinker region from pUC18.

pCGN566 contains the EcoRI to HindIII polylinker of pUC18 inserted into the EcoRI-HindIII sites of pUC13-Cm. The HindIII-BglII fragment of pNW31C-8,29-1 (Thomashow et al., Cell (1980) 19:729) containing ORF1 and -2 of pTiA6 was subcloned into the HindIII-BamHI sites of pCGN566 producing pCGN703.

The Sau3A fragment of pCGN703 containing the 3′ region of transcript 7 (corresponding to bases 2396-2920 of pTiA6 (Barker et al., (1983) supra) was sub-cloned into the BamHI site of pUC18 producing pCGN709. The EcoRI-SmaI polylinker region of pCGN709 was substituted with the EcoRI-SmaI fragment of pCGN587, which contains the kanamycin resistance gene (APH3-II) producing pCGN726.

The EcoRI-Sa1I fragment of pCGN726 plus the BglII-EcoRI fragment of pCGN734 were inserted into the BamHI-SalI site of pUC8-Cm producing pCGN738. pCGN726c is derived from pCGN738 by deleting the 900 bp EcoRI-EcoRI fragment.

To construct pCGN167, the AluI fragment of CaMV (bp 7144-7735) (Gardner et al., (1981) supra) was obtained by digestion with AluI and cloned into the HincII site of M13mp7 (Messing, et al., Nucl. Acid Res. (1981) 9:309-321) to create C614. An EcoRI digest of C614 produced the EcoRI fragment from C614 containing the 35S promoter which was cloned into the EcoRI site of pUC8 (Vieira and Messing et al., Gene (1982) 19:259) to produce pCGN146.

To trim the promoter region, the BglII site (bp 7670) was treated with BglII and resected with Bal31 and subsequently a BglII linker was attached to the Bal31 treated DNA to produce pCGN147.

pCGN148a containing a promoter region, selectable marker (KAN with 2 ATG's) and 3′ region, was prepared by digesting pCGN528 with BglII and inserting the BamHI-BglII promoter fragment from pCGN147. This fragment was cloned into the BglII site of pCGN528 so that the BglII site was proximal to the kanamycin gene of pCGN528.

The shuttle vector used for this construct, pCGN528, was made as follows. pCGN525 was made by digesting a plasmid containing Tn5 which harbors a kanamycin gene (Jorgenson et al., Mol. gen. Genet. (1979) 177:65) with HindIII-BamHI and inserting the HindIII-BamHI fragment containing the kanamycin gene into the HindIII-BamHI sites in the tetracycline gene of pACYC184 (Chang & Cohen, J. Bacteriol. (1978) 134:1141-1156). pCGN526 was made by inserting the BamHI fragment 19 of pTiA6 (Thomashow et al., Cell (1980) 19:729-739), modified with XhoI linkers inserted into the SmaI site, into the BamHI site of pCGN525. pCGN528 was obtained by deleting the small XhoI fragment from pCGN526 by digesting with XhoI and religating.

pCGN149a was made by cloning the BamHI-kanamycin gene fragment from pMB9KanXXI into the BamHI site of pCGN148a.

pMB9KanXXI is a pUC4K variant (Vieira and Messing, Gene (1982) 19:259-268) which has the XhoI site missing but contains a functional kanamycin gene from Tn903 to allow for efficient selection in Agrobacterium.

pCGN149a was digested with BglII and SphI. This small BglII-SphI fragment of pCGN149a was replaced with the BamHI-SphI fragment from MI (see below) isolated by digestion with BamHI and SphI. This produces pCGN167, a construct containing a full length CaMV promoter, 1ATG-kanamycin gene, 3′ end and the bacterial Tn903-type kanamycin gene. MI is an EcoRI fragment from pCGN546X (see construction of pCGN587) and was cloned into the EcoRI cloning site of M13 mp9 in such a way that the PstI site in the 1ATG-kanamycin gene was proximal to the polylinker region of M13mp9.

The HindIII-BamHI fragment in the pCGN167 containing the CaMV-35S promoter, 1ATG-kanamycin gene and the BamHI-fragment 19 of pTiA6 was cloned into the BamHI-HindIII sites of pUC19 creating pCGN976. The 35S promoter and 3′ region from transcript 7 was developed by inserting a 0.7 kb HindIII-EcoRI fragment of pCGN976 (35S promoter) and the 0.5 kb EcoRI-SauI fragment of pCGN709 (transcript 7:3′) into the HindIII-SalI sites of pCGN566 creating pCGN766c.

The 0.7 kb HindIII-EcoRI fragment of pCGN766c (CaMV-35S promoter) was ligated to the 1.5 kb EcoRI-SalI in pCGN726c (1ATG-KAN 3′ region) into the HindIII-SalI sites of pUC119 to produce pCGN778. The 2.2 kb region of pCGN778, HindIII-SalI fragment containing the CaMV-35S promoter and 1ATG-KAN-3′ region was used to replace the HindIII-SalI linker region of pCGN739 to produce pCGN783.

pCGN948 was introduced into Agrobacterium tumefaciens EHA101 (Hood et al, J. Bacteriol. (1986) 168:1291-1301) by transformation. An overnight two ml culture of EHA101 was grown in MG/L broth at 30°C. 0.5 ml was inoculated into 100 ml of MG/L broth (Garfinkel and Nester, J. Bacteriol. (1980) 144:732-743) and grown in a shaking incubator for 5 h at 30°C. The cells were pelleted by centrifugation at 7K, resuspended in 1 ml of MG/L broth and placed on ice. Approximately one µg of pCGN948 DNA was placed in 100 µl of MG/L broth to which 200 µl of the EHA101 suspension was added; the tube containing the DNA-cell mix was immediately placed into a dry ice/ethanol bath for 5 minutes. The tube was quick thawed by 5 minutes in a 37°C water bath followed by 2h of shaking at 30°C after adding 1 ml of fresh MG/L medium. The cells were pelleted and spread onto MG/L plates (1.5% agar) containing 100 mg/l gentamicin. Plasmid DNA was isolated from individual genta micin-resistant colonies, transformed back into E.coli, and characterized by restriction enzyme analysis to verify that the gentamicin-resistant EHA101 contained intact copies of pCGN948. Single colonies are picked and purified by two more streakings on MG/L plates containing 100 mg/l gentamicin.

Seeds of Brassica napus of Westar were soaked in 95% ethanol for 4 minutes. They were sterilized in 1% solution of sodium hypochlorite with 50 µl of "Tween 20" surfactant per 100 ml sterilent solution. After soaking for 45 minutes, seeds were rinsed 4 times with sterile distilled water. They were planted in sterile plastic boxes 7 cm wide, 7 cm long, and 10 cm high (Magenta) containing 50 ml of 10/10th concentration of MS (Murashige minimal organics medium, Gibco) with added pyridoxine (50 µg/l), nicotinic acid (50 µg/l), glycine

(200 µg/l) and solidified with 0.6% agar. The seeds germinated and were grown at 22°C in a 16h-8h light-dark cycle with light intensity approximately 65 $\mu Em^{-2}s^{-1}$. After 5 days, the seedlings were taken under sterile conditions and the hypocotyls excised and cut into pieces of about 4 mm in length. The hypocotyl segments were placed on a feeder plat or without the feeder layer on top of a filter paper on the solidified B5 0/1/1 or B5 0/1/0 medium. B5 0/1/0 medium contains B5 salts and vitamins (Gamborg, Miller and Ojima, Experimental Cell Res. (1968) 50:151-158), 3% sucrose, 2,4-dilchlorophenoxyacetic acid (1.0 mg/l), pH adjusted to 5.8, and the medium is solidified with 0.6% Phytagar; B5 0/1/1 is the same with the addition of 1.0 mg/l kinetin. Feeder plates were prepared 24 hours in advance by pipetting 1.0 ml of a stationary phase tobacco suspension culture (maintained as described in Fillatti et al., Mol. gen. Genet. (1987) 206:192-199) onto B5 0/1/0 or B5 0/1/1 medium. Hypocotyl segments were cut and placed on feeder plates 24 hours prior to Agrobacterium treatment.

Agrobacterium tumefaciens (strain EHA101 x 948) were prepared by incubating a single colony of Agrobacterium in MG/L broth at 30°C. Bacteria were harvested 16 hours later and dilutions of $10^8$ bacteria per ml were prepared in MG/L broth. Hypocotyl segments were inoculated with bacteria by placing in Agrobacterium suspension and allowed to sit for 30-60 minutes, then removed and transferred to Petri plates containing B5 0/1/1 or 0/1/0 medium described above. The plates were incubated in low light at 22°C. The co-incubation of bacteria with the hypocotyl segments took place for 24-48 hours. The hypocotyl segments were removed and placed on B5 0/1/1 or 0/1/0 containing 500 mg/1 carbenicillin (kanamycin sulfate at 10, 25, or 50 mg/l was sometimes added at this time) for 7 days in continuous light (approximately 65 $\mu Em^{-2}S^{-1}$) at 22°C. They were transferred to B5 salts medium containing 1% sucrose, 3 mg/1 benzylamino-purine and 1 mg/1 zeatin. This was supplemented with 500 mg/1 carbenicillin, 10, 25, or 50 mg/1 kanamycin sulfate, and solidified with 0.6% Phytagar (Gibco). Thereafter explants were transferred to fresh medium every 2 weeks.

After 1 month green shoots developed from green calli which were selected on media containing kanamycin. Shoots continued to develop for 3 months. The shoots were cut from the calli when they were at least 1 cm high and placed on B5 medium with 1% sucrose, no added growth substances, 300 mg/1 carbenicillin, and solidified with 0.6% phytagar. The shoots continued to grow and several leaves were removed to test for neomycin phosphotransferase II (NPTII) activity. Shoots which were positive for NPTII activity were placed in Magenta boxes containing B5 0/1/1 medium with 1% sucrose, 2 mg/1 indolebutyric acid, 200 mg/1 carbenicillin, and solidified with 0.6% Phytagar. After a few weeks the shoots developed roots and were transferred to soil. The plants were grown in a growth chamber at 22°C in a 16-8 hours light-dark cycle with light intensity 220 $\mu Em^{-2}s^{-1}$ and after several weeks were transferred to the greenhouse.

SOUTHERN DATA

Regenerated B. napus plants from cocultivations of Agrobacterium tumefaciens EHA101 containing pCGN948 and B. napus hypocotyls were examined for proper integration and embryo-specific expression of the spinach leaf ACP gene. Southern analysis was performed using DNA isolated from leaves of regenerated plants by the method of Dellaporta et al. (Plant Mol. Biol. Rep. (1983) 1:19-21) and purified once by banding in CsCl. DNA (10 µg) was digested with the restriction enzyme EcoR1, electrophoresed on a 0.7% agarose gel and blotted to nitrocellulose (see Maniatis et al., 1982, supra). Blots were probed with pCGN945 DNA containing 1.8 kb of the spinach ACP sequence or with the EcoRI/HindIII fragment isolated from pCGN936c (made by transferring the HindIII/EcoRI fragment of pCGN930 into pCGN566) containing the napin 5′ sequences labeled with $^{32}$P-dCTP by nick translation (described by the manufacturer, BRL Nick Translation Reagent Kit, Bethesda Research Laboratories, Gaithersburg, MD.). Blots were prehybridized and hybridized in 50% formamide, 10xDenhardt's, 5xSSC, 0.1% SDS, 5mM EDTA, 100µg/ml calf thymus DNA and d10% dextran sulfate (hybridization only) at 42°C. (Reagents described in Maniatis et al., (1982) supra). Washes were in 1xSSC, 0.1% SDS, 30 min and twice in 0.1xSSC, 0.1% SDS at 55°C.

Autoradiograms showed two bands of approximately 3.3 and 3.2 kb hybridized in the EcoRI digests of DNA from four plants when probed with the ACP gene (pCGN945) indicating proper integration of the spinach leaf ACP construct in the plant genome since 3.3 and 3.2 kb EcoRI fragments are present in the T-DNA region of pCGN948. The gene construct was present in single or multiple loci in the different plants as judged by the number of plant DNA-construct DNA border fragments detected when probed with the napin 5′ sequences.

NORTHERN DATA

Expression of the integrated spinach leaf ACP gene from the napin promoter was detected by Northern analysis in seeds but not leaves of one of the transformed plants shown to contain the construct DNA. Developing seeds were collected from the transformed plant 21 days post-anthesis. Embryos were dissected from the seeds and frozen in liquid nitrogen. Total RNA was isolated from the seed embryos and from leaves of the transformed plant by the method of Crouch et al. (1983) supra), electrophoresed on formaldehyde-containing 1.5% agarose gels as described (Shewmaker et al., Virology (1985) 140:281-288) and blotted to nitro-cellulose (Thomas, Proc. Natl. Acad. Sci., U.S.A. (1980) 77:5201-5205). Blots were prehybridized, hybridized and washed as described above. The probe was an isolated PstI/BamHI fragment from pCGN945 containing only spinach leaf ACP sequences labeled by nick translation.

An RNA band of ~0.8 kb was detected in embryos but not leaves of the transformed plant indicating seed-specific expression of the spinach leaf ACP gene.

Although higher plant fatty acid biosynthetic genes have been shown to recognize acyl-ACP substrates

when the ACP moiety is an Escherichia coli ACP, it remains possible that different forms of ACP from different sources may affect the efficiency and/or the final products of fatty acid synthesis in vivo. For instance, both spinach (Ohlrogge and Kuo, . Biol. Chem. (1985) 260:8032-8037) and barley (Hoj and Svendsen, Carlsberg. Res. Commun. (1984) 49:483-492, contain more than one form of ACP. To support the generality of using isoforms of ACP to enhance the value of oilseed crops, a cDNA copy of the gene for an ACP found in the seeds of the oilseed crop turnip rape (Brassica campestris) was isolated and characterized.

The following is the procedure for integrating the ACP gene into a chimeric gene using a napin promoter from Brassica campestris rather than Brassica napus.

Immature seeds are collected from Brassica campestris cv. "R-500", a self-compatible variety of turnip rape. Whole seeds are collected at stages corresponding approximately to 14 to 28 days after flowering. RNA isolation and preparation of a cDNA bank was as described above for the isolation of a spinach ACP cDNA clone. To probe the cDNA bank, the oligonucleotide (5')-ACTTTCTCAACTGTCTCTGGTTTAGCAGC-(3) was synthesized using an Applied Biosystems DNA Synthesizer, model 380A, according to manufacturer's recommendations. This synthetic DNA molecule will hybridize at low stringencies to DNA or RNA sequences coding for the amino acid sequence (ala-ala-lys-pro-glu-thr-val-glu-lys-val). This amino acid sequence has been reported for ACP isolated from the seeds of Brassica napus (Slabas et al., 7th International Symposium of the Structure and Function of Plant Lipids, University of California, Davis, CA, Plenum Press, N.Y. 1987); the ACP from B. campestrisseed is highly homologous. Approximately 2200 different cDNA clones are analyzed using a colony hybridization technique (Taub and Thompson, Anal. Biochem. (1982) 126:222-230) and hybridization conditions corresponding to (Wood et al., Proc. Natl. Acad. Sci. U.S.A. (1985) 82:1585-1588). DNA sequence anaysis of two cDNA clones showing obvious hybridization to the oligonucleotide probe indicated that one, designated pCGN1Bcs, coded for an ACP-precursor protein as evidenced by the considerable homology of the encoded amino acid sequence with ACP proteins described from Brassica napus (Slabas et al., supra). The DNA sequence of pCGN1Bcs (referred to also as AGB1) is indicated as follows:

AGB1 CDNA

```
                                    Tth111I
                                        TaqI
      XhoI                             SalI
       TaqI  Sau3AI                   HincII
      AvaI   BglII   AvaI      TaqI     AccI
      ||     |       |         |    | |||
  1 TCTCGAGCAGATCTCTCTCGGGAATATCGACAATGTCGACCACTTTCTGCTCTTCCGTCTCCATGCAAG   69
                                    METSerThrThrPheCysSerSerValSerMETGlnA
      3      10      18        28       37
       4     10                       35
      3                                36
                                    33

                                                AluI
                                                 |
 70 CCACTTCTCTGGCAGCAACAACGAGGATTAGTTTCCAGAAGCCAGCTTTGGTTTCAACGACTAATCTCT 138
    laThrSerLeuAlaAlaThrThrArgIleSerPheGlnLysProAlaLeuValSerThrThrAsnLeuS
                                                 115

                                                    HhaI
                                                       HaeIII
                                                    |  |
139 CCTTCAACCTCCGCCGTTCAATCCCCACTCGTTTCTCAATCTCCTGCGCGGCCAAACCAGAGACGGTTG 207
    erPheAsnLeuArgArgSerIleProThrArgPheSerIleSerCysAlaAlaLysProGluThrValG
                                                       190
                                                    187

              DdeI               AluI
              |                  |
208 AGAAAGTGTCTAAGATAGTTAAGAAGCAGCTATCACTCAAAGACGACCAAAAGGTCGTTGCGGAGACCA 276
    luLysValSerLysIleValLysLysGlnLeuSerLeuLysAspAspGlnLysValValAlaGluThrL
              218                237

            Sau3AI       HinfI  TaqI
            |            |      |
277 AGTTTGCTGATCTTGGAGCAGATTCTCTCGACACTGTTGAGATAGTGATGGGTTTAGAGGAAGAGTTTG 345
    ysPheAlaAspLeuGlyAlaAspSerLeuAspThrValGluIleValMETGlyLeuGluGluGluPheA
            285          298    305

        TaqI              DdeI
      EcoRV              AluI                       AluI
      | |                ||                         |
346 ATATCGAAATGGCTGAAGAGAAAGCTCAGAAGATTGCTACTGTGGAGGAAGCTGCTGAACTCATTGAAG 414
    spIleGluMETAlaGluGluLysAlaGlnLysIleAlaThrValGluGluAlaAlaGluLeuIleGluG
      348                370                        397
        350               371
```

```
    SacI
  AluI
  | |
415 AGCTCGTTCAACTTAAGAAGTAATTTTAGTATTAAGAGCAGCCAAGGCTTTGTTGGGTTTGTTGTTTTC 483
    luLeuValGlnLeuLysLys .
  417
    419


                                                        HinfI        DraI
                                                        |            |
484 ATAATCTTCCTGTCATTTTCTTTTTCTTTAATGTGTCAAGCGACTCTGTTGGTTTAAAGTAGTATCTGT 552
                                                        526          539

553 TTGCCAAAAAAA 564
```

To achieve high-level embryo-specific expression of a Brassica campestris seed ACP in a transgenic Brassica napus, a chimeric gene is made analogous to the embryo-specific chimeric gene employing spinach ACP-coding DNA sequences as described for pCGN946 above. The pCGN1Bcs ACP-coding region is adapted to fit into the Brassica campestris napin-type promoter element present in pCGN1803 (described below).

CONSTRUCTION OF B. CAMPESTRIS NAPIN PROMOTER CASSETTE

A BglII-partial genomic library of B. campestris DNA was made in the lambda vector Charon 35 using established protocols (Miniatis et al., (1982) supra). The titer of the amplified library was ~$1.2 \times 10^9$ phage/ml. Four hundred thousand recombinant bactriophage were plated at a density of $10^5$ per 9x9 NZY plate (NZYM as described in Maniatis et al., 1982 supra) in NZY + 10mM $MgSO_4$ + 0.9% agarose after adsorption to DH1 E. coli cells (Hanahan, D., J. Mol. Biol. (1983) 166:557) for 20 min at 37°C. Plates were incubated at 37°C for ~13 hours, cooled at 4°C for two and one half hours and phage were lifted onto GeneScreen Plus (New England Nuclear) by laying precut filters over the plates for approximately 1 min and peeling them off. The adsorbed phage DNA was immobilized by floating the filter on 1.5M NaCl, 0.5M NaOH for 1 min., neutralizing in 1.5M NaCl, 0.5M Tris-HCl, pH 8.0 for 2 min and 2XSSC for 3 min. Filters were air dried until just damp, prehybridized and hybridized at 42°C as described for Southern analysis. Filters were probed for napin-containing clones using an XhoI/SalI fragment of the cDNA clone BE5 which was isolated from the B. campestris seed cDNA library described above using the probe pN1 (Crouch et al., 1983. supra). Three plaques were hybridized strongly on duplicate filters and were plaque purified as described (Maniatis et al., 1982 supra).

The following is the BE5 cDNA sequence.

```
BE5 CDNA          LENGTH = 734

                               AluI
                               |
  1 ACAGAACATACACAAATGGCGAACAAGCTCTTCCTCGTCTCGGCAACTCTCGCCTTGTTCTTCCTTCTC  69
    ThrGluHisThrGlnMETAlaAsnLysLeuPheLeuValSerAlaThrLeuAlaLeuPhePheLeuLeu
                               28

                                       TaqI
                                      SalI                         NaeI
                                     HincII                       HpaII
                  AccI                 AccI                          HaeIII
                  |                  |||                          || |
 70 ACCAATGCCTCCGTCTACAGGACGGTTGTGGAAGTCGACGAAGATGATGCCACAAATCCAGCCGGCCCA 138
    ThrAsnAlaSerValTyrArgThrValValGluValAspGluAspAspAlaThrAsnProAlaGlyPro
                  84                   105                           135
                                     103                          132
                                      104                          133
                                       105

                                                      HindIII
                                                        AluI
                                                      | |
139 TTTAGGATTCCAAAATGTAGGAAGGAGTTTCAGCAAGCACAACACCTGAAAGCTTGCCAACAATGGCTC 207
    PheArgIleProLysCysArgLysGluPheGlnGlnAlaGlnHisLeuLysAlaCysGlnGlnTrpLeu
                                                        191
                                                      189

                                          AvaII
                        HpaII   AvaII  AluI     TaqI
                        |       |      |  |     |
208 CACAAGCAGGCAATGCAGTCCGGTAGTGGTCCAAGCTGGACCCTCGATGGTGAGTTTGATTTTGAAGAC 276
    HisLysGlnAlaMETGlnSerGlySerGlyProSerTrpThrLeuAspGlyGluPheAspPheGluAsp
                        228     236    243      252
                                          246

                                                                        SacI
                           HaeIII       HaeIII                        AluI
                           |            |                             | |
277 GACGTGGAGAACCAACAACAGGGCCCGCAGCAGAGGCCACCGCTGCTCCAGCAGTGCTGCAACGAGCTC 345
    AspValGluAsnGlnGlnGlnGlyProGlnGlnArgProProLeuLeuGlnGlnCysCysAsnGluLeu
                           300          313                           343
                                                                        345
        BstNI
        |
346 CACCAGGAAGAGCCACTTTGCGTTTGCCCAACCTTGAAAGGAGCATCCAAAGCCGTTAAACAACAGATT 414
    HisGlnGluGluProLeuCysValCysProThrLeuLysGlyAlaSerLysAlaValLysGlnGlnIle
        350
```

```
    TaqI
    |
415 CGACAACAACAGGGACAACAAATGCAGGGACAGCAGATGCAGCAAGTGATTAGCCGTATCTACCAGACC 483
    ArgGlnGlnGlnGlyGlnGlnMETGlnGlyGlnGlnMETGlnGlnValIleSerArgIleTyrGlnThr
    415

                       AluI                                            BstNI
                       |                                               |
484 GCTACGCACTTACCTAGAGCTTGCAACATCAGGCAAGTTAGCATTTGCCCCTTCCAGAAGACCATGCCT 552
    AlaThrHisLeuProArgAlaCysAsnIleArgGlnValSerIleCysProPheGlnLysThrMETPro
                       503                                             552

                                            XhoI
       HpaII                                 TaqI
    HaeIII                                  AvaI         AccI
      | |                                   ||           |
553 GGGCCCGGCTTCTACTAGATTCCAAACGAATATCCTCGAGAGTGTGTATACCACGGTGATATGAGTGTG 621
    GlyProGlyPheTyr .
       556                                  588          600
         558                                 589
                                            588

                 HincII                                        RsaI
                 |                                             |
622 GTTGTTGATGTATGTTAACACTACATAGTCATGGTGTGTGTTCCATAAATAATGTACTAATGTAATAAG 690

                 635                                           677

               AccI                         DraI
               |                            |
691 AACTACTCCGTAGACGGTAATAAAAGAGAAGTTTTTTTTTTAA 734

               702                          733
```

One of the clones named lambda CGN1-2 was restriction mapped, and the napin gene was localized to overlapping 2.7 kb XhoI and 2.1 kb SalI restriction fragments. The two fragments were subcloned from lambda CGN1-2 DNA into pCGN789 (a pUC based vector the same as pUC119 with the normal polylinker replaced by the synthetic linker - 5' GGAATTCGTCGACAGATCTCTGCAGCTCGAGGGATCCAAGCTT 3'-which represents the polylinker EcoRI, SalI, BglII, PstI, XhoI, BamHI, HindIII). The identity of the subclones as napin was confirmed by sequencing. The entire coding region sequence as well as extensive 5' upstream and 3' downstream sequences were determined.

Lambda CGN1-2

NCG-186 Linear          LENGTH = 4325

```
      XhoI
       TaqI                                                      HindIII
      AvaI                                                         AluI          TaqI
      ||                                                         | |             |
  1 CTCGAGGCAGTCACTAACATGAAGTTTGACGAGGAGCCCAACTATGGGAAGCTTATTTCTCTTTTCGAT   69

      2                                                            52            66
       3                                                         50
      2


                                                                   SacI
                        HhaI  XbaI                               AluI
                        |     |                                  | |
 70 ACTCTAATTGAGCCGTGCGCTCTATCTAGACCAATTAGAATTGATGGAGCTCTAAAGGTTGCTGGCTGT   138

                        89    95                                 119
                                                                   121


               NdeI                                                      NdeI
               |                                                         |
139 TTTCTTGTTCATATGATTAACTTCTAAACTTGTGTATAAATATTCTCTGAAAGTGCTTCTTTTGGCATA   207

               150                                                       206


208 TGTAGGTTGGGCAAAAACGAGGAAGATTGCTTCTCAATTTGGAAGAGGATGAACAGCCGAAGAAGAAAA   276


                                Sau3AI
                                    DdeI
                                |   |
277 TAAGAATAGGCAGTCCTGCTACTCAATGGATCTCAGTCTATAACGGTCGTCGTCCCATGAAACAGAGGT   345

                                    309
                                305


                                                                        EcoRV
                                                                        |
346 AAAACATTTTTTGCATATACACTTTGAAAGTTCCTCACTAACTGTGTAATCTTTTGGTAGATATCACTA   414

                                                                        408
```

```
                           HincII
                             HhaI
                            HaeIII
                            DdeI
                           BstEII
                            BalI                          HaeIII      AluI
                           |||                            |           |
415 CAATGTCGGAGAGACAA3GGCTGMNCANCATATACAAAAGGGAAATGAAGATGGCCTTTTGATTAGCTG  483

                            439                           469         481
                           438
                            439
                            439
                             440
                           438


                  AluI                                 HinfI
                  |                                    |
484 TGTAGCATCAGCAGCTAATCTCTGGGCTCTCATCATGGATGCTGGAACTGGATTCACTTCTCAAGTTTA  552

                  498                                  535


               MspI
               HpaII                                     HinfI
               |                                         |
553 TGAGTTGTCACCGGTCTTCCTACACAAGGTAATAATCAGTTGAAGCAATTAAGAATCAATTTGATTTGT  621

               564                                       606
               564


           DdeI
           |
622 AGTAAACTAAGAAGAACTTACCTTATGTTTTCCCCGCAGGACTGGATTATGGAACAATGGAAAAGAAC  690

           629


                                            SacI
               AluI    AluI               AluI
               |       |                  | |
691 TACTATATAAGCTCCATAGCTGGTTCAGATAACGGGAGCTCTTTAGTTGTTATGTCAAAAGGTTAGTGT  759

               702     710                729
                                            731


760 TTAGTGAATAATAAACTTATACCACAAAGTCTTCATTGACTTATTTATATACTTGTTGTGAATTGCTAG  828


                 DdeI                   HinfI
                 |                      |
829 GAACTACTTATTCTCAGCAGTCATACAAAGTGAGTGACTCATTTCCGTTCAAGTGGATAAATAAGAAAT  897

                 842                    865
```

XmnI TaqI

898 GGAAAGAAGATTTTCATGTAACCTCCATGACAACTGCTGGTAATCGTTGGGGTGTGGTAATGTCGAGGA 966

908 961

Sau3AI
BclI

967 ACTCTGGCTTCTCTGATCAGGTAGGTTTTTGTCTCTTATTGTCTGGTGTTTTTATTTTCCCCTGATAGT 1035

981
981

AluI RsaI

1036 CTAATATGATAAACTCTGCGTTGTGAAAGGTGGTGGAGCTTGACTTTTTGTACCCAAGCGATGGGATAC 1104

1074 1087

Sau3AI AluI

1105 ATAGGAGGTGGGAGAATGGGTATAGAATAACATCAATGGCAGCAACTGCGGATCAAGCAGCTTTCATAT 1173

1155 1165

ScaI
HinfI RsaI

1174 TAAGCATACCAAAGCGTAAGATGGTGGATGAAACTCAAGAGACTCTCCGCACCACCGCCTTTCCAAGTA 1242

1215 1242
1242

AluI Sau3AI DdeI

1243 CTCATGTCAAGGTTGGTTTCTTTAGCTTTGAACACAGATTTGGATCTTTTTGTTTTGTTTCCATATACT 1311

1268 1285 1311

DdeI
AvaII AluI HinfI RsaI

1312 TAGGACCTGAGAGCTTTTGGTTGATTTTTTTTTCAGGACAAATGGGCGAAGAATCTGTACATTGCATCA 1380

1315 1325 1363 1370
1319

1381 ATATGCTATGGCAGGACAGTGTGCTGATACACACTTAAGCATCATGTGGAAAGCCAAAGACAATTGGAG 1449

```
         HinfI
           DdeI
         | |
1450 CGAGACTCAGGGTCGTCATAATACCAATCAAAGACGTAAAACCAGACGCAACCTCTTTGGTTGAATGTA 1518

           1456
         1454


                                  RsaI
                                  |
1519 ATGAAAGGATGTGTCTTGGTATGTATGTACGAATAACAAAAGAGAAGATGGAATTAGTAGTAGAAATA 1587

                                  1548


             AluI                                      EcoRV
             |                                         |
1588 TTTGGGAGCTTTTTAAGCCCTTCAAGTGTGCTTTTTATCTTATTGATATCATCCATTTGCGTTGTTTAA 1656

             1596                                      1635


            XbaI                   DdeI
            |                      |
1657 TGCGTCTCTAGATATGTTCCTATATCTTTCTCAGTGTCTGATAAGTGAAATGTGAGAAAACCATACCAA 1725

            1664                   1687


                                        HinfI
                                        |
1726 ACCAAAATATTCAAATCTTATTTTTAATAATGTTGAATCACTCGGAGTTGCCACCTTCTGTGCCAATTG 1794

                                        1761


          HinfI                                                     EcoRI
          |                                                         |
1795 TGCTGAATCTATCACACTAGAAAAAAACATTTCTTCAAGGTAATGACTTGTGGACTATGTTCTGAATTC 1863

          1800                                                      1859


1864 TCATTAAGTTTTTATTTTCTGAAGTTTAAGTTTTTACCTTCTGTTTTGAAATATATCGTTCATAAGATG 1932


                                           SphI
            BstNI     AluI                   Sau3AI
            |         |                    | |
1933 TCACGCCAGGACATGAGCTACACATCGCACATAGCATGCAGATCAGGACGATTTGTCACTCACTTCAAA 2001

            1940      1950                   1973
                                           1971
```

```
                                               SphI
         DdeI  AluI            HhaI    NdeI  NsiI          Sau3AI
         |     |               |       |     | |           |
2002 CACCTAAGAGCTTCTCTCTCACAGCGCACACACATATGCATGCAATATTTACACGTGATCGCCATGCAA 2070

         2006  2012            2028    2036  2042          2058
                                               2044


2071 ATCTCCATTCTCACCTATAAATTAGAGCCTCGGCTTCACTCTTTACTCAAACCAAAACTCATCACTACA 2139


                             AluI
                             |
2140 GAACATACACAAATGGCGAACAAGCTCTTCCTCGTCTCGGCAACTCTCGGCCTTGTTCTTCCTTCTCACC 2208
                 METAlaAsnLysLeuPheLeuValSerAlaThrLeuAlaLeuPhePheLeuLeuThr
                             2164


                                     TaqI                          NaeI
                                    SalI                          MspI
                                   HincII                         HpaII
                AccI                 AccI                            HaeIII
                |                  |||                            || |
2209 AATGCCTCCGTCTACAGGACGGTTGTGGAAGTCGACGAAGATGATGCCACAAATCCAGCCGGCGCATTT 2277
     AsnAlaSerValTyrArgThrValValGluValAspGluAspAspAlaThrAsnProAlaGlyProPhe
                2220                 2241                            2271
                                   2239                           2268
                                    2240                          2268
                                     2241                          2269


                                                   HindIII
        HinfI                                        AluI
        |                                          | |
2278 AGGATTCCAAAATGTAGGAAGGAGTTTCAGCAAGCACAACACCTGAAAGCTTGCGAAGAATGGCTCCAC 2346
     ArgIleProLysCysArgLysGluPheGlnGlnAlaGlnHisLeuLysAlaCysGlnGlnTrpLeuHis
        2281                                         2327
                                                   2325


                      MspI               AvaII
                      HpaII   AvaII  AluI      TaqI
                      |       |      |  |      |
2347 AAGCAGGCAATGCAGTCCGGTAGTGGTCCAAGCTGGACCCTCGATGGTGAGTTTGATTTTGAAGACGAC 2415
     LysGlnAlaMETGlnSerGlySerGlyProSerTrpThrLeuAspGlyGluPheAspPheGluAspAsp
                      2364    2372   2379      2388
                      2364               2382
```

```
                         HaeIII                                       SacI
                           ApaI       HaeIII                        AluI
                         | |          |                             | |
2416 GTGGAGAACCAACAACAGGGCCCGCAGCAGAGGCCACCGCTGCTCCAGCAGTGCTGCAACGAGCTCCAC 2484
     ValGluAsnGlnGlnGlnGlyProGlnGlnArgProProLeuLeuGlnGlnCysCysAsnGluLeuHis
                           2438       2449                          2479
                         2436                                         2481

                                                                       TaqI
      BstNI                                                         HinfI
      |                                                             |  |
2485 CAGGAAGAGCCACTTTGCGTTTGCCCAACCTTGAAAGGAGCATCCAAAGCCGTTAAACAACAGATTCGA 2553
     GlnGluGluProLeuCysValCysProThrLeuLysGlyAlaSerLysAlaValLysGlnGlnIleArg
      2486                                                          2548
                                                                       2551

2554 CAACAACAGGGACAACAAATGCAGGGACAGCAGATGCAGCAAGTGATTAGCCGTATCTACCAGACCGCT 2622
     GlnGlnGlnGlyGlnGlnMETGlnGlyGlnGlnMETGlnGlnValIleSerArgIleTyrGlnThrAla

                     AluI                                             BstNI
                     |                                                |
2623 ACGCACTTACCTAGAGCTTGCAACATCAGGCAAGTTAGCATTTGCCCCTTCCAGAAGACCATGCCTGGG 2691
     ThrHisLeuProArgAlaCysAsnIleArgGlnValSerIleCysProPheGlnLysThrMETProGly
                     2639                                             2688

       MspI
       HpaII                         XhoI
     HaeIII                           TaqI
       ApaI         HinfI            AvaI        AccI
     | |            |                ||          |
2692 CCCGGCTTCTACTAGATTCCAAACGAATATCCTCGAGAGTGTGTATACCACGGTGATATGAGTGTGGTT 2760
     ProGlyPheTyr ,
       2694         2707             2724        2736
     2692                             2725
       2694                          2724
       2694

               HincII                                    RsaI
               |                                         |
2761 GTTGATGTATGTTAACACTACATAGTCATGGTGTGTGTTCCATAAATAATGTACTAATGTAATAAGAAC 2829

               2771                                      2813

             AccI
             |
2830 TACTCCGTAGACGGTAATAAAAGAGAAGTTTTTTTTTTTACTCTTGCTACTTTCCTATAAAGTGATGAT 2898

             2838
```

```
                                                                             ScaI
                                                                             RsaI
                                                                             |
2899 TAACAACAGATACACCAAAAAGAAAACAATTAATCTATATTCACAATGAAGCAGTACTAGTCTATTGAA 2967

                                                                             2954
                                                                             2954

                                                                                  Sau3AI
                                                                                  |
2968 CATGTCAGATTTTCTTTTTCTAAATGTCTAATTAAGCCTTCAAGGCTAGTGATGATAAAAGATCATCCA 3036

                                                                                  3028

         Sau3AI                     Sau3AI
         BamHI       HinfI          BclI
         |           |              |
3037 ATGGGATCCAACAAAGACTCAAATCTGGTTTTGATCAGATACTTCAAAACTATTTTTGTATTCATTAAA 3105

         3041        3053           3069
         3041                       3069

                                 HinfI
                                 |
3106 TTATGCAAGTGTTCTTTTATTTGGTGAAGACTCTTTAGAAGCAAAGAACGACAAGCAGTAATAAAAAAA 3174

                                 3135

3175 ACAAAGTTCAGTTTTAAGATTTGTTATTGACTTATTGTCATTTGAAAAATATAGTATGATATTAATATA 3243

3244 GTTTTATTTATATAATGCTTGTCTATTCAAGATTTGAGAACATTAATATGATACTGTCCACATATCCAA 3312

                               NdeI
                               |
3313 TATATTAAGTTTCATTTCTGTTCAAACATATGATAAGATGGTCAAATGATTATGAGTTTTGTTATTTAC 3381

                               3341

                        TaqI                 Sau3AI
                     AluI                RsaI
                     |  |                |   |
3382 CTGAAGAAAAGATAAGTGAGCTTCGAGTTTCTGAAGGGTACGTGATCTTCATTTCTTGGCTAAAAGCGA 3450

                     3402                3421
                        3405                 3425

3451 ATATGACATCACCTAGAGAAAGCCGATAATAGTAAACTCTGTTCTTGGTTTTTGGTTTAATCAAACCGA 3519
```

```
                                                                 MspI
      MspI  DdeI                                                 HpaII
      HpaII AluI                               NdeI            HinfI
      |     ||                                 |               |    |
3520 ACCGGTAGCTGAGTGTCAAGTCAGCAAACATCGCAAACCATATGTCAATTCGTTAGATTCCCGGTTTAA 3588

      3522  3528                               3560            3576
      3522   3529                                                   3581
                                                                    3581


             MspI
             HpaII
             |
3589 GTTGTAAACCGGTATTTCATTTGGTGAAAACCCTAGAAGCCAGCCANCCTTTTTAATCTAATTTTTGCA 3657

             3598
             3598


                                                               HinfI
                                                                HincII
                                                   DdeI            BstNI
                                                   |           ||  |
3658 AACGAGAAGTCACCACACCTCTCCACTAAAACCCTGAACCTTACTGAGAGAAGCAGAGNCANNAAAGAA 3726

                                                   3702            3718
                                                                3715
                                                               3714


3727 CAAATAAAACCCGAAGATGAGACCACCACGTGCGGCGGGACGTTCAGGGGACGGGGAGGAAGAGAATGR 3795


      AvaII
         AluI                                                         AvaII
      |  |                                                            |
3796 CGGCGG5MNTTTGGTGGCGGCGGCGGACGTTTTGGTGGCGGCGGTGGACGTTTTGGTGGCGGCGGTGGA 3864

         3804                                                         3863
      3801


               EcoRV       AvaII                                    DdeI
               |           |                                        |
3865 CCTTTGGTGGTGGATATCGTGACGAAGGACCTCCCAGTGAAGTCATTGGTTCGTTTACTCTTTTCTTAG 3933

               3880        3892                                     3930
```

```
     TaqI                                    HindIII
       HinfI                                   AluI                   DdeI
     | |                                     | |                       |
3934 TCGAATCTTATTCTTGCTCTGCTCGTTGTTTTACCGATAAAGCTTAAGACTTTATTGATAAAGTTCTCA 4002

       3937                                    3976                    4000
     3935                                    3974


     AluI                XmnI                                    HinfI     DdeI
     |                   |                                       |         |
4003 GCTTTGAATGTGAATGAACTGTTTCCTGCTTATTAGTGTTCCTTTGTTTTGAGTTGAATCACTGTCTTA 4071

     4004                4023                                    4059      4069


                  HinfI
                  |
4072 GCACTTTTGTTAGATTCATCTTTGTGTTTAAGTTAAAAGGTAGAAACTTTGTGACTTGTCTCCGTTATG 4140

                  4085


          HincII
          |
4141 ACAAGGTTAACTTTGTTGGTTATAACAGAAGTTGCGACCTTTCTCCATGCTTGTGAGGGTGATGCTGTG 4209

          4146


     AvaII  AluI DdeI     Sau3AI
     |      |    |        |
4210 GACCAAGCTCTCTCAGGCGAAGATCCCTTACTTCAATGCCCCAATCTACTTGGAAAACAAGACACAGAT 4278

     4210   4217 4222     4231


                                          TaqI
                                         SalI
                                       PstI
                          HindIII      HincII
                    Sau3AI  AluI         AccI EcoRI
                    |     | |          ||||    |
4279 TGGGAAAGTTGATGAGATCCAAGCTTGGGCTGCAGGTCGACGAATTC 4325

                    4294    4302          4316 4321
                          4300
                                        4314
                                       4313
                                         4315
                                          4316
```

The lambda CGN1-2 napin gene is that encoding the mRNA corresponding to the BE5 cDNA as determined by the exact match of their nucleotide sequences.

An expression cassette was constructed from the 5'-end and 3'-end of the lambda CGN1-2 napin gene as follows in an analogous manner to the construction of pCGN944. The majority of the napin coding region of pCGN940 was deleted by digestion with SalI and religation to form pCGN1800. Single-stranded DNA from pCGN1800 was used in an in vitro mutagenesis reaction (Adelman et al., DNA (1983) 2:183-193) using the synthetic oligonucleotide 5'-GCTTGTTCGCCATGGATACTTGTGTATGTTC-3'. This oligonucleotide inserted an EcoRV and an NcoI restriction site at the junction of the promoter region and the ATG start codon of the napin gene. An appropriate mutant was identified by hybridization to the oligonucleotide used for the mutagenesis and sequence analysis and named pCGN1801.

A 1.7 kb promoter fragment was subcloned from pCGN1801 by partial digestion with EcoRV and ligation to pCGN786 (a pCGN566 chloramphenicol based vector with synthetic linker described above in place of the normal polylinker) cut with EcoRI and blunted by filling in with DNA Polymerase I Klenow fragment to create pCGN1802. 3' sequences from the lambda CGN1-2 napin gene were added to XhoI/HindIII digested pCGN1802 to complete the cassette by ligation to pCGN941 digested with XhoI and HindIII. The resulting expression cassette, pCGN1803 contains 1.725 kb of napin promoter sequence, and 1.265 kb of napin 3' sequences with the unique cloning sites SalI, BglII, PstI and XhoI in between.

The ACP-precursor coding region from pCGN1Bcs is excised by double digestion with Xho1 and EcoRI and ligated to the cloning vector pUC18 previously digested with SalI and EcoRI. Transformation of ligated DNA into the appropriate E. coli host and screening using the penicillin-resistant, blue-white screening system of pUC vectors (Vieira and Messing, Gene (1982) 19:259-268) generates a plasmid containing a unique Dra3 site located downstream of the stop codon for the ACP-precursor coding region. Digestion with Dra3 followed by ligation with a PstI linker generates a plasmid in which the coding region is cleanly excised as a BglII to PstI fragment, which is cloned into PstI and BglII sites of pCGN1803 with the proper orientation of the ACP-precursor coding region to the napin promoter and terminator parts of the embryo-specific expression information encoded by pCGN1803. The resulting chimeric gene is then introduced into a binary vector and transferred to Agrobacterium for cocultivation with Brassica napus hypocotyl segments employing the same conditions employed for the spinach ACP chimeric gene in pCGN946.

In accordance with the subject invention, sequences coding for functional acyl carrier protein, particularly plant acyl carrier protein, are provided, which can be used as probes for detecting the presence of acyl carrier protein genes, for screening libraries from plants and bacteria, either genomic or cDNA, for use in assays for detecting the presence of acyl carrier protein genes, and the like. In addition, the coding sequence can be used in the preparation of expression constructs, where the coding sequence is combined with transcriptional and translational initiation and termination regulatory regions for expression in an appropriate host in which the regulatory regions are functional. A particular interest is the use of the ACP coding sequence in conjunction with a transcription initiation region which is functional in a plant and particularly is regulated so as to provide for expression in seed. In this manner, the production of seed oil can be enhanced and, as appropriate, the fatty acid composition modulated.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

## Claims

1. A cDNA sequence coding for plant acyl carrier protein.
2. A cDNA sequence according to Claim 1, wherein said plant is spinach.
3. A cDNA sequence according to Claim 1, wherein said plant is Brassica.
4. A cDNA sequence according to Claim 3, wherein said Brassica is campestris.
5. A cDNA sequence according to Claim 3, wherein said Brassica is napus.
6. A DNA construct comprising an open reading frame coding for a plant acyl carrier protein joined to and under transcriptional and translational initiation and termination regulatory regions functional in a cellular host, wherein at least one of said regulatory regions is other than the wild-type region of said open reading frame.
7. A DNA construct according to Claim 6, wherein said open reading frame encodes for spinach or Brassica acyl carrier protein.
8. A DNA construct according to Claim 6, wherein said regulatory regions are functional in a plant host.
9. A DNA construct according to Claim 8, wherein said transcriptional initiation region is regulated during cell differentiation.
10. A DNA construct according to Claim 9, wherein said regulation provides for transcription in the developing embryo.
11. A DNA construct according to Claim 10, wherein said transcriptional initiation regulatory region is a napin regulatory region.
12. A Ti- or Ri-plasmid comprising a DNA construct according to any of Claims 6 to 11.

13. A plant cell comprising a DNA construct according to any of Claims 6 to 11.

14. A plant cell according to Claim 13, wherein said plant cell is an embryonic cell and part of a seed.

15. A plant cell according to Claim 14, wherein said plant is a Brassica plant.

16. A method for enhancing the formation of vegetable seed oils which comprises,
growing a plant from plantlet to seed, wherein the cells of said plant comprise a construct according to Claim 8, and
harvesting the resulting seed containing an enhanced amount of vegetable oil.

17. A method according to Claim 16, wherein said plant is a Brassica plant.

18. A method according to any of Claims 16 or 17, wherein said transcriptional initiation regulatory region is a napin region.